# EUROPEAN PATENT SPECIFICATION

(11) **EP 3 031 904 B1**
(45) Date of publication and mention of the grant of the patent: **06.06.2018**
(21) Application number: 14197677.9
(22) Date of filing: 12.12.2014
(51) Int. Cl.: C12N 1/12, C12R 1/89, A61K 36/02, A23L 5/00, A23L 33/10, C12M 1/00

(54) **COMPOSITION COMPRISING SALT AND MICROALGAE BIOMASS, PROCESS OF ITS PRODUCTION AND USES (WITH VARIANTS)**
ZUSAMMENSETZUNG MIT SALZ UND MIKROALGENBIOMASSE, VERFAHREN ZU DEREN HERSTELLUNG UND VERWENDUNGEN (MIT VARIANTEN)
COMPOSITION COMPRENANT SEL ET BIOMASSE DE MICRO-ALGUES, SON PROCÉDÉ DE PRODUCTION ET UTILISATIONS (AVEC DES VARIANTES)

(43) Date of publication of application: 15.06.2016
(73) Proprietor: Omarov, Farhad, Bornova, Izmir (TR)
(72) Inventor: OMAROV, Farhad, Izmir (TR); OMRAOV, Sindbad, Izmir (TR); ALI-ZADE, Mukhtar, 7323 NZ Apeldoorn (NL); ALI-ZADE, Murad, 7323 NZ Apeldoorn (NL)
(74) Representative: Glawe, Delfs, Moll

(56) References cited:
- WO-A1-2010/105095
- US-A1- 2012 225 472
- PAULA DÍAZ PALMA ET AL: "Biochemical profile of halophilous microalgae strains from high-andean extreme ecosystems (NE-Chile) using methodological validation approaches", JOURNAL OF BIOSCIENCE AND BIOENGINEERING, ELSEVIER, AMSTERDAM, NL, vol. 113, no. 6, 25 January 2012 (2012-01-25), pages 730-736, XP028483130, ISSN: 1389-1723, DOI: 10.1016/J.JBIOSC.2012.01.015 [retrieved on 2012-02-03]
- KH TAMBIEV A ET AL: "Manifestation of salt tolerance ofandcyanobacteria of the genus", MOSCOW UNIVERSITY BIOLOGICAL SCIENCES BULLETIN, ALLERTON PRESS, INC, HEIDELBERG, vol. 66, no. 4, 21 December 2011 (2011-12-21), pages 133-137, XP019990496, ISSN: 1934-791X, DOI: 10.3103/S0096392511040079
- HEMAISWARYA S ET AL: "Microalgae: a sustainable feed source for aquaculture", WORLD JOURNAL OF MICROBIOLOGY AND BIOTECHNOLOGY, KLUWER ACADEMIC PUBLISHERS, DO, vol. 27, no. 8, 24 December 2010 (2010-12-24), pages 1737-1746, XP019929017, ISSN: 1573-0972, DOI: 10.1007/S11274-010-0632-Z

## Description

### FIELD OF THE INVENTION

The present invention relates to processes of preparation of compositions comprising sodium chloride and sodium chloride resistant microalgae biomass, said process comprising: a) providing: i) a carrier comprising sodium chloride; ii) a living sodium chloride resistant microalgae biomass; b) mixing said living salt resistant microalgae biomass with said carrier; c) drying said mixture. The present invention further relates to compositions produced by the processes of the present invention, their multiple uses and novel products based thereon.

### BACKGROUND OF THE INVENTION

*Spirulina* (also known as *Arthrospira)* is a type of free-floating, non-flagellated, non-cyst-forming blue-green algae with a soft cell wall made up of proteins and polysaccharides. It appears as a blue-green filaments composed of cylindrical cells arranged in e.g. un-branched, helicoi-dally trichomes. Its cells have a variable cell length and width (e.g. 1 - 20 µm) and length/width ratio (e.g. 0.1 - 10), e.g. a cell can have a length of about 1 µm (micrometer) and a width of about 1-2 µm (micrometer). It has no true nucleus. It shows gliding motility of filamentous cells (trichomes) with rotation along their long axis. It is rich in protein, vitamins (e.g. vitamins A, B1, B2, B6, B12, C, E and H), minerals, carotenoids, phycocyanin, chlorophyll A and other antioxidants that can help protect cells from damage (e.g. oxidative). It has no bacteriorhodopsin. It contains multiple nutrients, including B-complex vitamins, beta-carotene, vitamin E, manganese, zinc, copper, iron, selenium, and essential fatty acids (e.g. gamma linolenic acid and others) and therefore can be used in prevention and treatment of vitamin deficiency and other disorders (e.g. malnutrition, anemia). The blue-green algae, e.g. *Spirulina,* have a primitive structure with few starch storage cells and cell membrane proliferation, but rich amounts of ribosomes, the cellular bodies that manufacture protein. This particular arrangement of cellular components allows for rapid photosynthesis and formation of proteins. The lack of rigid cellular walls assures that *Spirulina* protein is rapidly and easily assimilated by any consuming organisms (its protein cell wall can be dissolved). It has an approximately 65 to 71% of protein, depending on growing conditions. These proteins are biologically complete, which means that they provide all eight essential amino acids in the necessary ratios. Furthermore, it provides all the required amino acids in a form that is easier to digest than meat or soy protein. *Spirulina* is an ideal anti-aging food. It concentrates great nutrient value, load with antioxidants and easily digested and therefore it can be used as an antioxidant. *Spirulina* is also rich in iron, magnesium and trace minerals that essential for the body, but yet is the most common mineral deficiency in daily ration. *Spirulina* is the highest source of B-12, essential for healthy nerves and tissue, e.g. for vegetarians. About 60% of *Spirulina*'s dry weight is protein, which is essential for growth and cell regeneration. Its chemical content can be influenced by growing conditions. It also stimulates beneficial gut flora, e.g. bacteria of genus *Lactobacillus* (e.g. *Lactobacillus acidophilus*), bacteria of genus *Bifidobacterium (e.g. Bifidobacterium bifidum)* in digestive tract promoting healthy digestion and proper bowel function (e.g. it can be used as a prebiotic). It acts as a natural cleanser by eliminating mercury and other deadly toxins commonly ingested by the body and therefore it can also be used as a detoxifying agent. *Chlorella* is a genus of single-cell non-motile, non-flagellated, non-cyst-forming green microalgae, which has a rigid cell wall comprising cellulose (e.g. the cell wall of Chlorella is composed of up to 80% carbohydrates including cellulose). Its cells have spherical shape, about 2-10 micrometers (µM) in diameter and have a nucleus. Its chemical content can be influenced by growing conditions. *Chlorella* comprises green photosynthetic pigments chlorophyll A and B, vitamin C and carotenoids (e.g. beta-carotene), all of which are antioxidants, but it has no bacteriorhodopsin. Antioxidants are compounds that block the action of free radicals (unstable molecules that can damage cells). *Chlorella* is also reported to contain high concentrations of iron and B-complex vitamins. *Chlorella* cells (e.g. cells of *Chlorella pyrenoidosa* and/or *Chlorella vulgaris)* also comprise an antibacterial substance chlorellin, which is reported to have bacteriostatic and bactericidal activity against microorganisms (e.g. gram-positive microorganisms). *Chlorella* cells comprise essential fatty acids (e.g. arachidonic acid), and are considered to be an effective producer of arachidonic acid. *Chlorella* cells (e.g. *Chlorella pyrenoidosa*) have been reported to comprise an acidic polysaccharide Chlon A that has immune enhancing and anti-tumor effects and stimulate interferon biosynthesis. *Chlorella* is also a known herbal treatment for a wide range of conditions and diseases. It is claimed to fight bacterial and viral infections, enhances the immune system, increases the growth of "friendly" germs in the digestive tract (e.g. prebiotic), lower blood pressure and cholesterol levels, and promote healing of intestinal ulcers (e.g. duodenal ulcer, gastrojejunal ulcer), diverticulosis, and Crohn's disease. Through its ability to cleanse the body of toxins and heavy metals and due to its property to increase the level of albumin (protein that normally present in the bloodstream) in the body, *Chlorella* helps to protect the organism against diseases such as cancer, diabetes, arthritis, AIDS, pancreatitis, cirrhosis, hepatitis, anemia, and multiple sclerosis and can also be used in prevention and treatment of vitamin deficiency. However, the use of dried microalgae biomass-based products (e.g. *Spirulina-* and *Chlorella*-based) is hindered by high degradability of numerous biologically active cellular components/ substances/compounds during the process of drying or extracting. Thus, drying or extracting the biomass leads to e.g. cell destruction, disruption of cellular structures and complexes, and corresponding decrease in the content/concentration of valuable nutrients and associated activities of the microalgae biomass. It also leads to changes in ratios of nutrients and also changes chemical structure (e.g. oxidation status) of cellular components/substances/compounds. Therefore, there is a need for processes and compositions that combine a high-degree preservation of biologically active cellular components/substances/compounds of the microalgae biomass with availability and transportability of dry biomass-based compositions.

### SUMMARY OF THE INVENTION

The present invention is defined in the claims and provides a process of preparation of a composition comprising sodium chloride and sodium chloride resistant microalgae biomass, said process comprising:
a) providing: i) a carrier comprising sodium chloride, preferably said carrier is selected from the group consisting of: table salt, rock salt, refined salt, lake salt, sea salt and Himalayan salt; ii) a living sodium chloride resistant microalgae biomass selected from the group consisting of: *Arthrospira (Spirulina) maxima* designated strain Absheron Accession No. BEA D03_12, *Chlorella vulgaris* designated strain Novruz 2012 Accession No. BEA D02_12, and mixtures thereof, preferably said living sodium chloride resistant microalgae biomass is freshly grown, further preferably said living sodium chloride resistant microalgae biomass is freshly grown on a growing medium comprising said carrier as a source of sodium chloride, and b) mixing said living sodium chloride resistant microalgae biomass with said carrier, said mixing comprises at least three-stages, wherein each following stage has a greater sodium chloride concentration than a previous stage; c) drying said mixture, preferably said drying is carried out under light protection conditions, further preferably said light protection conditions have illuminance of less than 1000 lux, and wherein at least 75% of cells of said sodium chloride resistant microalgae biomass remain whole after drying. In another embodiment the present invention provides a process as described above, wherein said living sodium chloride resistant microalgae biomass is grown on a growing medium comprising said carrier as a source of sodium chloride. In another embodiment the present invention provides a process as described above, wherein 99% of cells of said sodium chloride resistant microalgae biomass remain whole after drying. In another example of the present specification the following process of preparation of a composition comprising sodium chloride and sodium chloride resistant microalgae biomass is suggested: a) providing living sodium chloride resistant microalgae biomass; b)adding sodium chloride to said living sodium chloride resistant microalgae biomass and mixing said living salt resistant microalgae biomass with sodium chloride, preferably said adding and mixing comprises at least three-stages, wherein each following stage has an increased sodium chloride concentration compared to a previous stage, further preferably said three-stage mixing has a proportion to each successive step of 1/10, 1/10 and 1/100; c) drying the mixture, preferably said drying is carried out under a light protection conditions. The present invention provides a process as described above and claimed in claim 1, wherein said composition has one or more of the following features: cells of said sodium chloride resistant microalgae biomass are non-flagellated; cells of said sodium chloride resistant microalgae biomass are non-cyst-forming; cells of said sodium chloride resistant microalgae biomass are non-motile, preferably said non-motile cells are free-floating; cells of said sodium chloride resistant microalgae biomass have a cell wall, preferably a rigid cell wall, further preferably a rigid cell wall comprising cellulose; said sodium chloride resistant microalgae biomass is capable of growing at sodium chloride concentration in the range of 0.1 - 1 M, preferably in the range of 0.1 - 0.75 M; further preferably in the range of 0.1 - 0.5 M; said sodium chloride resistant microalgae biomass is capable of growing in the temperature range of 0°C - 45°C, preferably 10°C - 45°C, further preferably in the temperature range of 20°C - 45°C; said sodium chloride resistant microalgae biomass is capable of growing in the pH range of 6.0-14.0 or in the pH range of 5.0-9.0; after drying said sodium chloride resistant microalgae biomass has a total concentration of polyunsaturated fatty acids of at least 2% of its dry weight; after drying said sodium chloride resistant microalgae biomass has a concentration in said composition of about 1-2% of dry weight; said living sodium chloride resistant microalgae biomass has a productivity of at least 5 g/m², preferably at least 10 g/m² further preferably at least 20 g/m², further preferably at least 40 g/m², further preferably at least 80 g/m²; said sodium chloride resistant microalgae biomass comprises a pigment chlorophyll, preferably said chlorophyll is chlorophyll A; said sodium chloride resistant microalgae biomass comprises an antibacterial substance chlorellin; said sodium chloride resistant microalgae biomass comprises phycocyanin; said sodium chloride resistant microalgae biomass comprises arachidonic acid (ARA), preferably said biomass is a producer of arachidonic acid (ARA); said sodium chloride resistant microalgae biomass comprises an acidic polysaccharide Chlon A; cells of said sodium chloride resistant microalgae biomass have no nuclei; said microalgae biomass does not comprise bacteriorhodopsin. In another embodiment the present invention provides a composition comprising sodium chloride and sodium chloride resistant microalgae biomass produced by the process of claims 1-4 and as described above. In another embodiment the present invention provides a dry composition comprising: a) a carrier comprising sodium chloride, preferably said carrier is selected from the group consisting of: table salt, rock salt, refined salt, lake salt, sea salt and Himalayan salt, and b) a sodium chloride resistant microalgae biomass selected from the group consisting of: *Arthrospira (Spirulina) maxima* designated strain Absheron Accession No. BEA D03_12, *Chlorella vulgaris* designated strain Novruz 2012 Accession No. BEA D02_12, and mixtures thereof, wherein at least 75% of cells of said sodium chloride resistant microalgae biomass remain whole in said composition. In another embodiment the present invention provides a composition as described above, wherein said composition has one or more of the following features: cells of said sodium chloride resistant microalgae biomass are non-flagellated; cells of said sodium chloride resistant microalgae biomass are non-cyst-forming; cells of said sodium chloride resistant microalgae biomass are non-motile, preferably said non-motile cells are free-floating; cells of said sodium chloride resistant microalgae biomass have a cell wall, preferably a rigid cell wall, further preferably a rigid cell wall comprising cellulose; said sodium chloride resistant microalgae biomass is capable of growing at sodium chloride concentration in the range of 0.1 - 1 M, preferably in the range of 0.1 - 0.75 M; further preferably in the range of 0.1 - 0.5 M; said sodium chloride resistant microalgae biomass is capable of growing in the temperature range of 0°C - 45°C, preferably 10°C - 45°C, preferably in the temperature range of 20°C - 45°C; said sodium chloride resistant microalgae biomass is capable of growing in the pH range of 6.0-14.0 or in the pH range of 5.0-9.0; after drying said sodium chloride resistant microalgae biomass has a total concentration of polyunsaturated fatty acids of at least 2% of its dry weight; after drying said sodium chloride resistant microalgae biomass has a concentration in said composition of about 1-2% of dry weight; said living sodium chloride resistant microalgae biomass has a productivity of at least 5 g/m², preferably at least 10 g/m² further preferably at least 20 g/m², further preferably at least 40 g/m², further preferably at least 80 g/m²; said sodium chloride resistant microalgae biomass comprises a pigment chlorophyll, preferably said chlorophyll is chlorophyll A; said sodium chloride resistant microalgae biomass comprises an antibacterial substance chlorellin; said sodium chloride resistant microalgae biomass comprises phycocyanin; said sodium chloride resistant microalgae biomass comprises arachidonic acid (ARA), preferably said biomass is a producer of arachidonic acid (ARA); said sodium chloride resistant microalgae biomass comprises an acidic polysaccharide Chlon A; cells of said sodium chloride resistant microalgae biomass have no nuclei; said microalgae biomass does not comprise bacteriorhodopsin.In another embodiment the present invention provides a composition as described above for use as a medicament. In yet another embodiment the present invention provides a composition as described above for use in prevention and/or treatment of a disease selected from the group consisting of: vitamin deficiency, iron-deficiency anemia, megaloblastic anemia, pernicious anemia, intestinal ulcers, diverticulosis, Crohn's disease, hypertension, cancer, diabetes, arthritis, AIDS, pancreatitis, cirrhosis, hepatitis, anemia, and multiple sclerosis. The present invention further provides use of a composition as described above in preparation of a foodstuff or a fodder, or an intermediate foodstuff or an intermediate fodder, or a nutritional supplement, or a prebiotic, or a pharmaceutical composition, or a seasoning, or a spice or a mixture thereof. The composition can also be used in the detoxification or antioxidant treatment as claimed in the enclosed claim set. The present invention also provides a foodstuff or a fodder, or an intermediate foodstuff or an intermediate fodder, or a nutritional supplement, or a prebiotic, or a pharmaceutical composition, or a seasoning, or a spice, or a detoxifying agent, or an antioxidant comprising a composition as described above. The present invention further provides non-flagellated, non-cyst-forming sodium chloride resistant microalgae, wherein said sodium chloride resistant microalgae is selected from the group consisting of: *Arthrospira (Spirulina) maxima* designated strain Absheron Accession No. BEA D03_12 and *Chlorella vulgaris* designated strain Novruz 2012 Accession No. BEA D02_12. In another embodiment the present invention provides processes, compositions, their uses and novel products based thereon as disclosed herein, wherein the language "comprising" is replaced with the language "consisting of". Any single embodiment of the present invention described herein can be combined with another single or multiple embodiment/s of the present invention described herein.

### DETAILED DESCRIPTION OF THE INVENTION

In the context of the present invention the term "sodium chloride resistant" means that corresponding biomass (e.g. cell or microorganism) can survive sodium chloride in their environment, wherein sodium chloride can be present at any concentration (e.g. anywhere in the range of about 0.05 - 1 M; 0.1 - 1 M, 0.1 - 0.75 M; 0.05 - 0.75 M; 0.1 - 0.5 M; 0.05 - 0.5 M; up to 1M, up to 0.75M, up to 0.5M, at least: 0.05M, or 0.1M or 0.2M or 0.3M or 0.4M or 0.5M or 0.6M or 0.7M or 0.8M or 0.9M or 1M). The term "about" means "approximately" (e.g. ±0.5). The term "biomass" means cells. The term "microalgae" means microscopic algae with cell sizes that can range from micrometer (pm) to a few hundreds of micrometers (e.g. 1-10, 1-20, 1-30, 1-40, 1-50, 1-100, 1-200, 1-300, 1-400, 1-500, 1-900 µm). The term "algae" means organisms that have chlorophyll as their primary photosynthetic pigment and lack a sterile covering of cells around their reproductive cell, said meaning of the term "algae" includes cyanobacteria. The term "carrier" means a substance or a composition. The term "living" means alive. The term "under light protection conditions" means less light than in the open environment (e.g. by the means of a barrier, e.g. shelter; light protection conditions can have illuminance of less than 1000 lux). The term "majority" means the number larger than half the total (e.g. 51%, 55%, 60%, 65%, 70%, 75%, 80%, 85%, 90% 95%, 99%, 100%). The term "non-flagellated" means without flagella. The term "non-cyst-forming" means not able to form a cyst. The term "polyunsaturated fatty acids" means fatty acids that contain more than one double bond in their backbone (e.g. omega-3 fatty acids, e.g. eicosapentaenoic acid, docosahexaenoic acid; omega-6 fatty acids, e.g. linoleic acid, gamma-linolenic acid, arachidonic acid). The term "an acidic polysaccharide Chlon A" means an extractable (e.g. by hot water extraction) acidic polysaccharide from *Chlorella* with immunomodulatory properties. The term "remain whole" means that corresponding cells remain whole (e.g. unbroken and/or undisrupted). The term "table salt" means refined salt containing about 97 to 99 percent sodium chloride. The term "rock salt" means halite, the mineral form of sodium chloride. The term "refined salt" means salt containing about 97 to 99 percent sodium chloride, which has one or more of the following features: it has been cleaned, re-crystallized, iodized, and sized. The term "lake salt" means salt produced from salt lake's water. The term "sea salt" means salt produced from seawater. The term "Himalayan salt" means halite comprising 95-96% sodium chloride. The term "foodstuff" means any substance used or capable of being used as nutriment. The term "fodder" means food for animals (e.g. birds, livestock, wild). The term "intermediate" means subject to further processing. The term "nutritional supplement" includes vitamins, minerals, herbs, meal supplements, sports nutrition products, natural food supplements, and other related products used to increase the nutritional content of the diet. The term "prebiotic" means nutrient that changes the composition and/or activity in the gastrointestinal microflora, conferring health benefits on the host. The term "seasoning" means a composition for heightening or improving the flavor of food. The term "spice" means pungent or aromatic substance used to flavor food. The term "antioxidant" means a composition protecting cells from the damaging effects of oxidation. The term "detoxifying agent" means a composition counteracting, destroying or removing the effects of toxic substance. The problem to be solved by the present invention can *inter alia* be seen in provision of a novel and improved process of preparation of a dried composition comprising microalgae biomass such that degradation of the cellular content of said biomass is decreased compared to known processes. As such the problem to be solved can also be seen in provision of a novel dry composition comprising microalgae biomass with increased functionality, including provision of suitable microalgae biomass strains. The problem is solved according to the claims of the present invention. Indeed, it has been established in the course of the present invention that mixing sodium chloride and living microalgae biomass prior to drying preserves the cellular content of said biomass. That is because in such a process cells of the microalgae remain whole after drying. In one embodiment the present invention provides a method of production of foodstuff for animals (e.g. livestock, agricultural, wildlife animals). Such animal feed salt products (e.g. based on rock salt, refined salt, lake salt, sea salt and Himalayan salt) comprise a salt (e.g. sodium chloride) with addition of the biomass of microalgae selected from the group consisting of: *Arthrospira (Spirulina) maxima* designated strain Absheron Accession No. BEA D03_12, *Chlorella vulgaris* designated strain Novruz 2012 Accession No. BEA D02_12, and mixtures thereof. Such salt compositions can be used e.g. as foodstuff, fodder or an intermediate fodder or as a nutritional supplement on farms, e.g. poultry farms (e.g. in chicken meat production, broiler production, production of eggs, goose meat production, production of duck meat, turkey meat production), on cattle farms (e.g. in production of dairy products, meat cows (e.g. beef), buffaloes, on pig farms (e.g. in pork meat production), for small livestock (e.g. goats and sheep), for horse keeping and/or breeding and on camels farms, and further for feeding animals in nature reserves and eco-parks (e.g. deer, elk, wild boar and etc.), including zoos. In another embodiment the present invention provides compositions (e.g. products designated as "Agri-ALGASALT" or "Animal-ALGASALT") that are in a granular form for addition to the feed mixtures (for example on the combifeed farms) and production of a second product type, e.g. a salt licks-stones e.g. for horses, cows, pigs, sheep, deer and others, which is designated as "ALGASALT-BLOCK". In another embodiment a method of manufacturing of "ALGASALT" product for the first time applies "live" concentrated biomass of microalgae *Arthrospira (Spirulina)* and/or *Chlorella* and/or mixtures thereof to create a new: foodstuff, fodder, intermediate foodstuff, intermediate fodder, nutritional supplement, prebiotic, pharmaceutical composition, seasoning, spice, detoxifying agent, antioxidant or mixture thereof. Over 2 years the authors of the present invention carried out scientific experiments to create a universal technology to provide the most complete preservation technology of the cellular content of microalgae *Spirulina (Arthrospira) platensis* and/or *maxima*) and/or *Chlorella* (e.g. *Chlorella vulgaris*) as described herein. Based on the above, a full "organic" basis of all "ALGASALT" production cycles can be produced. The present invention also provides *Chlorella vulgaris* designated strain Novruz 2012 (Accession No. BEA D02_12, deposited on March 19, 2012 with Spanish Bank of Algae (BEA), Marine Biotechnology Center, University of Las Palmas, Gran Canaria, Muelle de Taliarte s/n, 35214 Telde, Las Palmas; depositors: Farhad Omarov and Maryam Omarova address on the date of filing of the patent application: D 1, B-1 Blok, No 4/3, 119/2 Sok., Standart Kent Sitesi, Evka-3, Bornova, Iz-mir, Turkey) and *Arthrospira (Spirulina) maxima* designated strain Absheron (Accession No. BEA D03_12, deposited on March 19, 2012 with Spanish Bank of Algae (BEA) as above, same depositors, same address). In another embodiment the processes of the present invention are not multi-technological and easily amenable to automation. The process itself comprises (e.g. consists of) two main cycles: 1^{st} cycle - growing (getting the living biomass of microalgae *Spirulina* and/or *Chlorella*) and 2^{nd} cycle - process of mixing. Cultivation of microalgae biomass: microalgae *Chlorella vulgaris* (author's name "Buzovna") grow during the whole year. Industrial production of microalgae requires easily accessible raw materials: e.g. sunlight, water, carbon dioxide and nutrients (e.g. nitrogen, sources of phosphate and potassium, e.g. phosphates and potassium salts). In processes of the present invention a maximum light is used (e.g. a photo-bioreactor is used), which considerably increases the productivity. In one embodiment, the density of the resulting algal culture is 1 gram per 1 liter of suspension. In another embodiment of the present invention the biomass of *Spirulina* and/or *Chlorella* is grown in an open photo-bioreactor or pavilion (e.g. greenhouse). Implementation of processes of the invention can lead to space economy, e.g. photo-bioreactors can be mounted vertically, both indoors and outside in open areas. The reactor significantly reduces labor costs and eliminates the complexity of maintenance. The system can operate for long periods of time without failure of suspension culture. Photo-bioreactor equipped with comfortable self-cleaning system that reduces contamination can also be used. The reactor provides a closed, controlled and automated continuous conditions that allow easier maintenance of sanitary conditions and microbiological purity of cultures. Environmental parameters of the system is also easily managing. The size of the reactor is not limited. Used photo-bioreactors stimulate the rapid growth of algae. Moreover, proposed processes have no biological and/or chemical waste. Modern factory for the production of food salt, especially in the case of sea salt production by evaporation - can be upgraded for manufacturing compositions of the present invention. Engineering solution, e.g. a cascade/line of industrial baths (e.g. food plastic containers) can be provided, where processes of the present invention can be carried out, followed by drying, packaging, logistics and market. Compositions of the present invention are designated as "ALGASALT". In one embodiment the ALGASALT salt crystals acquire a beautiful green or blue-green tint. In another embodiment the process of the present invention comprises inspection and/or sieving of the carrier (e.g. sodium chloride crystals), mixing it with live (e.g. freshly grown) microalgae biomass, drying in special light protection baths, followed by wrapping and packing. In one embodiment the mixing can advantageously be carried out stepwise: e.g. not less than three stages, for example, in proportion to each successive step 1:10, 1:10 and 1:100. This allows a superior preservation of cellular content and therefore production of compositions with curative and protective (e.g. therapeutic and/or preventive) properties, e.g. a sodium chloride-based composition "ALGASALT". Therefore the higher the degree of preservation of cellular content the more effective is the use of the composition as described herein. Therefore, the compositions of the present invention have an omega-3 and omega-6 concentration almost 10 higher than that of the pink salt composition from the Sivash-lake comprising cyst-forming flagellated *Dunaliella* microalgae (which lacks a rigid cell wall and is enclosed by a thin cytoplasmic membrane only), almost all cells of which are disrupted and extracted in said pink salt composition, with concentration of *Dunaliella* biomass in said composition being up to 0.1% of dry mass (e.g. 0.01% dry mass). Therefore, even a pinch (less than 1 gram) of the blue-green salt ALGASALT is able to enrich food with chlorophyll, vitamins, minerals and other active substances, which are essential for normal functioning of the human body. Microalgae-derived Chlorophyll is an efficient absorbent and therefore blue-green salt ALGASALT, getting into the food system becomes a biological detoxifying agent. And natural antioxidants from living microalgal biomass protect people from oncological diseases. The compositions of the present invention are also advantageously affordable compared to other supplements.

### EXAMPLES

The present invention provides sodium chloride resistant *Chlorella vulgaris* designated strain Novruz 2012 Accession No. BEA D02_12 and sodium chloride resistant *Arthrospira (Spirulina) maxima* designated strain Absheron Accession No. BEA D03_12 and mixtures thereof.

### I. Chlorella vulgaris designated strain Novruz 2012 Accession No. BEA D02_12

### II.1 Characteristics of the strain

According to the algological classification system, the taxonomic position of *Chlorella vulgaris* strain of the present invention can be reflected by the following scheme: Kingdom - *Plantae;* Class - *Chlorophyceae;* Division - *Chlo-rophycota;* Order - *Chlorellales;* Family - *Chlorellaceae;* Genus - *Chlorella;* Species - *Chlorella vulgaris;* Strain - *Chlorella vulgaris Novruz 2012 BEA D02_12.*

This strain is a new thermophilic, salt-resistant strain of microscopic unicellular green algae with a wide range of industrial cultivation temperature (e.g. from 0°C - 45°C (growth at lower temperatures has decreased cell division rate), preferably from 10°C to 45°C, broad optimum from 20 °C to 35 °C, optimum 20°C - 30 °C: highest yield). This strain has an enhanced biomass accumulation, i.e. it is suitable for industrial cultivation.

Thermophilicity of this strain requires a high temperature optimum of +40°C for the production of biomass and an increased salt resistance allows the cells of this strain to withstand the sodium chloride concentration of up to 0.5 M NaCl (e.g. 29 g NaCl/liter or even 30 g NaCl/liter). Besides this strain withstands a wide range of acidity (pH) in the course of cultivation (e.g. pH from 5.0 to 9.0). Physiological and biochemical properties (in % of dry biomass) : protein 21 - 55% (depending on the mode of cultivation (growth); lipids (fat) 12%; carbohydrates 10.0 - 25.0%; ash content 7 - 8%. The strain has an extremely high plasticity of metabolism, i.e. it has an ability to radically change the direction of biosynthesis depending on culturing conditions and under different treatments. Carbohydrates may be represented by starch and predominantly by hemicelluloses. Lipids comprise 12-30% and are characterized by a considerable content of unsaturated fatty acids. The ratio of fatty acids in Chlorella is similar to the ratio, which is typical to most vegetable oils. The content of amino acids depends on the composition of culturing media (growth medium) and cultivation conditions, e.g. parametric control of directed synthesis is the following (g/kg of air-dry matter): Glutamic acid 30.0 - 38.0; Aspartic acid 20.0 - 25.0; Leucine 15.0 - 22.0; Alanine 20.0 - 24.0; Valine 16.0 - 17.0; Glycine 14.0 - 18.0; Threonine 12.0 - 15.5; Phenylalanine 11.0 - 12.0; Serine 11.0 - 12.0; Isoleucine 10.0 - 11.0; Proline 8.0 - 9.5, Lysine 7.0 - 8.0; Tyrosine 8.0 - 9.0; Arginine 6.0 - 9.0; Cystine 6.0 - 8.0; Tryptophan 5.0 - 6.0; Methionine 3.0 - 5.0; Histidine 1.0 - 3.0. In suspension of this strain a significant amount of vitamins is synthesized. Vitamin contents of Chlorella includes (µg (i.e. microgram)/g of dry matter): Beta-carotene (provitamin A) 1000.0 - 1300.0; Tocopherol (E) 150.0 - 180.0; Niacin 120.0 - 140.0; Riboflavin (B2) 6.0 - 7.0; Pyridoxine (B6) 4.0 - 6.0; Thiamine 3.0 - 5.0. This strain shows well-defined antagonistic properties to the algal flora, bacteria, fungi, yeast and infusoria. Bacteria die and precipitate on the bottom. This strain strictly complies with the terms and monoculture and is not sensitive to phages. This strain is highly productive and has an antibacterial ability.

### II. Arthrospira (Spirulina) maxima designated strain Absheron Accession No. BEA D03_12

### II.1 Classification

*Arthrospira maxima* is a filament planktonic cyanobacteria. However, according to the algological classification system, the taxonomic position of *Spirulina (Arthrospira) maxima* strain of the present invention can be reflected by the following scheme: Kingdom - *Prokaryotae;* Monera - *Bacteria;* Class - *Cyanophyceae;* Division - *Cyanophycota;* Order - *Nostocales;* Family - *Oscillatoriaceae;* Genus - *Arthrospira;* Species - *Arthrospira maxima;* Strain - *Arthrospira (Spirulina) maxima Absheron BEA D03_12.* According to the modern microbiological classification system the taxonomic position of the same strain can also be described by the following scheme: Kingdom - *Prokaryotae;* Domain - *Bacteria;* Division - *Gracilicutes;* Class - *Oxyphotobacteria;* Groups - *Cyanobacteria;* Order - *Oscillatoriales;* Genus - *Arthrospira;* Species - *Arthrospira maxima;* Strain - *Arthrospira (Spirulina) maxima Absheron BEA D03_12.*

### II.2 Characteristics of the strain

Physiological and biochemical properties: this strain does not fix atmospheric nitrogen, instead uses nitrates and ammonium as sources of nitrogen. As carbon sources uses bicarbonates (predominantly), carbonates, organic compounds (even glucose). The maximum biomass growth occurs in the sunlight. However, the biomass can also be grown with use of light sources of any spectral composition and under strongly shaded conditions. Optimum growth is observed with using natural sunlight. Photoperiod is not determined and there is no seasonality. With accumulation of biomass cell culture of this strain floats (surfaces), which is advantageous for industrial production of the biomass of this strain. This strain is sodium chloride resistant and grows in a salinity range of 0.1 to 1M NaCl. Optimum growth within the range of 0.35 - 0.5 M NaCl. Growth observed at pH ranging from 6.0 to 14.0; optimum at pH range of 9.5 to 10.5. The growth is thermophilic, i.e. the strain grows at temperatures ranging from 0°C - 45°C (growth at lower temperatures has decreased cell division rate), preferably from 20°C to 45°C; broad optimum from 20°C to 35 °C optimum in the range of 30°C to 35°C. Inhibition and destruction of the culture occurs at temperatures above 50°C. Depending on cultivation conditions it can have a productivity anywhere between 5 g/m² - 80 g/m² (e.g. 5, 10, 20, 30, 40, 50, 60, 70, 80). Chemical composition of cells depends on the growth conditions and the composition of the growing medium. Parametric control in the production of biomass provides a wide range of compositions (in % of dry biomass): protein 50.0-75.0; carbohydrates 12.0-20.0; fats (lipids) 5.0-7.0; nucleic acids 5.5-6.5; Chlorophyll (A) 0.9-0.95; ash content 7.0. Component g/100: Nitrogen 8-10; Phosphorus 1-1.1; Sodium 1-2; Potassium 1-2; Magnesium 0.2-0.3; Calcium 0.8-1.0; Sulfur 0.5-0.6. Component mg/kg: Iron 800-1900; Copper 11-15; Manganese 44-60; Nickel 3-5; Zinc 22-30; Cobalt 1-1.5; Chlorine is 2-5; Chrome 3-5; Iodine 500-100. The protein content in Spirulina is (60-70%), which is much higher than in any other traditional foods. For comparison, the egg contains 47% protein, beef - 18-21%, soybean powder - 37%. In addition, Spirulina protein contains all the necessary (essential) for the normal functioning of the human body amino acids. Its composition can include up to 8% of fat including essential fatty acids (e.g. total concentration of polyunsaturated fatty acids of died Spirulina is 2.08 g per 100 g), in particular, gamma-linolenic acid. And, most importantly, Spirulina has optimal ratios of essential vitamins, e.g. vitamins A, B1, B2, B3, B6, B12 and PP, biotin, folic acid, inositol, pantothenate as well as vitamins C and E. Very important is also the digestibility of Spirulina proteins by the body. Spirulina cell walls do not contain rigid cellulose and consist of mucopolysaccharides, which allows Spirulina proteins to be easily absorbed (up to 87%), which is very important for human and animal nutrition. Spirulina is rich in vitamins, e.g. Beta
- carotene (provitamin A), vitamins B12, B1, B2, B6, PP (niacin), E, Folacin (folic acid). Based on the content of vitamin B2 Spirulina is superior to any other animal product. Spirulina has almost all the necessary set of individual minerals, which are present in an easily digestible form. With a relatively low content of fat (e.g. 8%) Spirulina is rich in unsaturated fatty acids (vitamin F), especially in gamma-linolenic acid, linoleic, oleic, which are necessary for the synthesis of phospholipids - a main component of cell membranes and vascular walls; prostaglandins
- tissue hormones that control the regulation of blood pressure, neurotransmission, condition of the walls of blood vessels, liver metabolism and play an important role in lipid metabolism, prevention of atherosclerosis, regulation of reproductive functions of the human body. Carbohydrates are presented in Spirulina by polysaccharides (e.g. glycogen and others), which are easily absorbed by the body with minimal amount of insulin. The carbohydrate content of Spirulina is e.g. 10-20%. Spirulina contains three pigments: carotenoids, chlorophyll and phycocyanin, which help the body to synthesize many enzymes necessary for the regulation of the metabolism of the body. The most important of these is the blue-green pigment phycocyanin. Studies conducted by Japanese and American physicians, show that phycocyanin strengthens the immune system and improves lymphatic activity of the organism. The main function of the lymphatic system is to maintain healthy organs in the body and protect against cancer, ulcers, hemorrhoids and other diseases. Spirulina's chlorophyll has structure and chemical composition close to the heme molecule of blood. In combination with a set of substances contained in Spirulina, chlorophyll promotes the biosynthesis of hemoglobin, which allows to normalize the function of blood-forming organs in a short time. The presence of lipids, sulfolipids and enzymes in Spirulina increases the effects of Spirulina as an immuno-tonic composition.

### III. Processes of the present invention:

Exemplary process of preparation of a composition comprising sodium chloride and sodium chloride resistant microalgae biomass has been carried out within the present invention, said process comprising: a) providing: i) sodium chloride; ii) living sodium chloride resistant microalgae biomass, wherein said sodium chloride resistant microalgae biomass is selected from the group consisting of: *Arthrospira (Spirulina) maxima* designated strain Absheron Accession No. BEA D03_12, *Chlorella vulgaris* designated strain Novruz 2012 Accession No. BEA D02_12 and mixtures thereof, b) mixing said living salt resistant microalgae biomass with said sodium chloride, said mixing comprises three-stages, wherein each following stage has a greater sodium chloride concentration than a previous stage; c)
drying said mixture. The process of the pending invention allows the majority of cells of sodium resistant microalgae biomass (e.g. 100% or at least 99%, or at least 95%, or at least 90%, or at least 85%, or at least 80%, or at least 75%) to remain whole after drying (e.g. cells are not destructed and/or cells /cell structures are not disrupted and/or extracted), which advantageously distinguishes the process of the present invention from other processes based on mixing dried (i.e. dead) microalgae biomass and salt, which normally leads to up to 40% decrease in usefulness of the biomass (e.g. decrease in concentration and/or degradation and/or oxidation of biologically active components of the cell, e.g. compounds, complexes, vitamins, omega-3 and/or omega-6 fatty acids). Therefore, such other dried (i.e. dead) microalgae biomass-based processes lead to cell destruction and corresponding decrease in the content of valuable nutrients and associated activities of the microalgae biomass. Based on the technical effect of the provision of the living sodium chloride resistant microalgae biomass within the process of the present invention, the process step (c) of the process as described above, i.e. drying said mixture, provides the result in that the majority of cells of said sodium chloride resistant microalgae biomass remain whole after drying. In another example the three-stages mixing process is carried out as follow: in the first stage a microalgae biomass is added to sodium chloride in order to maximally, but softly increase its salinity threshold; then in the second stage salinity is further increased by addition of more sodium chloride to the mixture resulting from the first stage; finally, in the third stage a desired ratio of biomass to sodium chloride is obtained by addition of even more sodium chloride to the mixture resulting from the second stage. Therefore, in order to get 100g of the ALGASALT composition (i.e. composition comprising sodium chloride and sodium chloride resistant microalgae biomass) with 1% of dried biomass of *Arthrospira (Spirulina) maxima* designated strain Absheron Accession No. BEA D03_12, *Chlorella vulgaris* designated strain Novruz 2012 Accession No. BEA D02_12 or mixtures thereof (e.g. mixture consisting of 50% *Arthrospira* and 50% *Chlorella*), an initial 5g of raw microalgae biomass can be taken (e.g. consistency of the biomass can be described as "paste"); wherein from said 5g of raw microalgae biomass with "paste" consistency a 1g of dried biomass of *Arthrospira (Spirulina) maxima* or *Chlorella vulgaris* or mixtures thereof (e.g. 50/50 mixture) can be produced.

Therefore, the three-stage mixing can be described as follows: (a) first stage: (500 g of biomass + 500 g sodium chloride); (b) second stage: resulting mixture of (a) + 1kg sodium chloride; (c) resulting mixture of (b) + 8.4 kg sodium chloride. In this example 500g of raw biomass produce 100 g of dried biomass, to which 9900g sodium chloride has to be added resulting 10kg ALGASALT composition with 1% wgt of biomass. The product of the exemplary process described above (i.e. ALGASALT) is e.g. dry and, e.g. granular, light green or light blue-green crystals. Said crystals can be finely ground - with the planes of the crystals of about 0.1 - 0.25 mm. The crystals ALGASALT can also be larger e.g. with crystals planes of about 2.5 - 5.0 mm. In another example the process step (c) of the process as described above, i.e. drying said mixture, is carried out under light protection conditions, preferably said light protection conditions have illuminance of less than 1000 lux. This is advantageous e.g. in order to minimize the degradation process of valuable nutrients e.g. chlorophyll (e.g. chlorophyll A) and pigments of algae of *Spirulina* and *Chlorella.* In the course of the present invention it has been established that light adversely effects chlorophyll and other pigments in cells during drying (e.g. according to the process of the present invention). Therefore, salt baths, e.g. even on the large industrial scale (e.g. volume from 1 to 100 tons) can be advantageously provided with at least a shelter in step c) of the process. Furthermore, light can destroy Omega fatty acids derivatives. Therefore, protection from light (i.e. light protection conditions) have an additional function of preservation of Omega fatty acids bioactive components of microalgae biomass. In another example, a living sodium chloride resistant microalgae biomass from step a) ii) of the process as described above is freshly grown. In yet another example it is freshly grown on a growing medium comprising said carrier as a source of sodium chloride. In one example, a sea salt "Billur" (Turkey) is used as a carrier (e.g. for the production of ALGASALT). In that example Spirulina and/or Chlorella biomass of the present invention is grown on a nutrient medium, in which the carrier (e.g. sea salt "Billur") is used as a source of sodium chloride. In that example the final product ALGASALT comprises the sea salt "Billur" (Turkey) as a carrier, whereas the sea salt "Billur" is also comprised within the cells of Spirulina and/or Chlorella "Billur" (Turkey). In another example a basic substance (e.g. salt, sodium chloride) can be any salt, e.g. rock salt, refined salt, lake salt, sea salt and Himalayan salt, of any manufacturer (e.g. different manufacture/s, e.g. from different countries). The production process can also be carried out on any scale, e.g. 1kg, 5 tons, 80 tons.

### IV. Use of the compositions and processes of the present invention:

In another example the processes and compositions of the present invention comprise a unique technology of "live iron" enrichment of other products and methods e.g. a foodstuff or a fodder for e.g. enrichment of a diet, e.g. of a human diet, and/or agricultural and other animals (e.g. livestock and/or game and/or birds). Thus the compositions and processes of the present invention can be used e.g. for an enrichment (and/or regulation) of e.g. iron (Fe), and/or vitamins B2, B6, B12 content of a foodstuff or a fodder, or an intermediate foodstuff or an intermediate fodder, or a nutritional supplement, or a prebiotic, or a pharmaceutical composition, or a seasoning, or a spice, or a detoxifying agent, or an antioxidant or mixtures thereof. In another example Chlorophyll A comprised in compositions and processes of the present invention is used as a source of organic iron (e.g. a main source), especially if combined with other nutritional complexes contained in Spirulina and/or Chlorella cells (e.g. I/Fe/Se combination). Chlorophyll-derived iron is also easily digestible. Chlorophyll A promotes the biosynthesis of hemoglobin, which allows to normalize the function of blood-forming organs in a short time. Chlorophyll A is also involved in the detoxification of the body. A chlorophyll content of microalgae Spirulina and Chlorella is 3-5 times higher than that of Alfalfa (i.e. *Medicago sativa,* also called lucerne). In another example Spirulina comprises the following pigments and vitamins. Pigments, mg/100g, (%): Carotenoids 5.2 (0.015%); Chlorophyll A 50.0 (0.15%); Phycocyanins 500.0 (1.5). Furthermore, *Spirulina* has optimal ratios (i.e. for digestion and/or supplementation) of essential vitamins: e.g. vitamins A, C, E, PP, group B. Exemplary Spirulina vitamins are the following, with content in mg/kg, and function/importance in: Beta-carotene (provitamin A) 1700 (Organs of vision, reproduction, and immunity); B1 (thiamine) 55 (Peripheral and central nervous system); B2 (riboflavin) 24 (Protection the liver from toxic substances); B3 (niacin) 118 (Nutrition to the nerve cells); B5 (pantothenic acid) 11 (Exchange of fats, carbohydrates, amino acids, fatty acid synthesis, cholesterol); B6 (pyridoxine) 3 (Fat metabolism, haematopoiesis, central nervous system, a powerful antioxidant);

B8 (Inositol) 350 (Supports a "healthy" status of liver, clearing the body of toxins, heavy metals;, and normalizes cholesterol); B12 (cobalamin) 1.6 (Exchange fats, neurotransmission); Bc (folic acid) 0.5 (Biosynthesis of hemoglobin); PP (niacin) 118 (Elasticity of blood vessels, an antioxidant); E (tocopherol) 190 (Absorption of calcium and phosphorus, bone growth); C (ascorbic acid) 180 (Protein synthesis, energy production). Minerals (e.g. micro- and macronutrients) of Spirulina are powerful bio-regulators of normal development of the organism. They play a crucial biological role, e.g. by interacting with amino acids, pigments, vitamins, affecting the processes of hematopoiesis, respiratory of tissues, normalizing the activity of the cardiovascular, nervous and other systems of the body. One of the best examples of the Spirulina minerals is an organic iron. Due to the high content of biologically active iron Spirulina consumption is an effective way to increase hemoglobin in the treatment of iron deficiency and/or pernicious anemia. Based on its iron content Spirulina has a superior position (e.g. a first place) in the plant kingdom, and comprises 100 times greater amounts of iron than e.g. apples, or radishes, or cabbages or potatoes. Exemplary content of microelements in Spirulina is the following, in microgram/100g: selenium 6.0; copper 52.0; silicon (Si) 3100.0; chrome 11.0; cobalt 8.0; molybdenum 5.0; iodine 4.5-9.0; germanium 3.9. Exemplary content of macro-elements in Spirulina is the following, in microgram/kg: calcium 1200; magnesium 3700; boron 3000; phosphorus 8300; potassium 14000; sodium 300; iron 528; zinc 170; manganese 200; chlorine 4200. In another example 100 grams of algae Chlorella comprise: chlorophyll A - 1 mg; chlorophyll B - 613 mg; beta-carotene (provitamin A) - 180.8 mg; vitamin C - 15.6 mg; thiamine (vitamin B1) - 1.5 mg; riboflavin (vitamin B2) - 4.8 mg; pyridoxine (vitamin B6) - 1.7 mg; niacin (vitamin B3 or PP, or niacin) - 23.8 mg; pantothenic acid (vitamin B5) - 1.3 mg; folic acid (vitamin B9) - 26.9 mg; vitamin B12 - 125.9 microgram; biotin (vitamin B7 or vitamin H) - 191.6 mg; vitamin K - 165 mg; p-amino-benzoic acid (4-Aminobenzoic acid, PABA); phosphorus - 989 mg; magnesium - 315 mg; iron - 167 mg; calcium - 203 mg; copper - 0.08 mg; zinc - 71 mg;

## Claims

1. A process of preparation of a composition comprising sodium chloride and sodium chloride resistant microalgae biomass, said process comprising:
a) providing:
i) a carrier comprising sodium chloride;
ii) a living sodium chloride resistant microalgae biomass selected from the group consisting of: *Arthrospira (Spirulina) maxima* designated strain Absheron Accession No. BEA D03 12, *Chlorella vulgaris* designated strain Novruz 2012 Accession No. BEA D02_12, and mixtures thereof, preferably said living sodium chloride resistant microalgae biomass is freshly grown, further preferably said living sodium chloride resistant microalgae biomass is freshly grown on a growing medium comprising said carrier as a source of sodium chloride, and
b) mixing said living sodium chloride resistant microalgae biomass with said carrier, said mixing comprises at least three-stages, wherein each following stage has a greater sodium chloride concentration than a previous stage;
c) drying said mixture, preferably said drying is carried out under light protection conditions, further preferably said light protection conditions have illuminance of less than 1000 lux, and wherein at least 75% of cells of said sodium chloride resistant microalgae biomass remain whole after drying.

2. The process of claim 1, wherein said living sodium chloride resistant microalgae biomass is grown on a growing medium comprising said carrier as a source of sodium chloride.

3. The process of claims 1-2, wherein 99% of cells of said sodium chloride resistant microalgae biomass remain whole after drying.

4. The process of claims 1-3, wherein said composition has one or more of the following features:
a. cells of said sodium chloride resistant microalgae biomass are non-flagellated;
b. cells of said sodium chloride resistant microalgae biomass are non-cyst-forming;
c. cells of said sodium chloride resistant microalgae biomass are non-motile, preferably said non-motile cells are free-floating;
d. cells of said sodium chloride resistant microalgae biomass have a cell wall, preferably a rigid cell wall, further preferably a rigid cell wall comprising cellulose;
e. said sodium chloride resistant microalgae biomass is capable of growing at sodium chloride concentration in the range of 0.1 - 1 M, preferably in the range of 0.1 - 0.75 M; further preferably in the range of 0.1 - 0.5 M;
f. said sodium chloride resistant microalgae biomass is capable of growing in the temperature range of 0°C - 45°C, preferably 10°C - 45°C, further preferably in the temperature range of 20°C - 45°C;
g. said sodium chloride resistant microalgae biomass is capable of growing in the pH range of 6.0-14.0 or in the pH range of 5.0-9.0;
h. after drying said sodium chloride resistant microalgae biomass has a total concentration of polyunsaturated fatty acids of at least 2% of its dry weight;
i. after drying said sodium chloride resistant microalgae biomass has a concentration in said composition of 1-2% of dry weight;
j. said living sodium chloride resistant microalgae biomass has a productivity of at least 5 g/m², preferably at least 10 g/m² further preferably at least 20 g/ m², further preferably at least 40 g/m², further preferably at least 80 g/m²;
k. said sodium chloride resistant microalgae biomass comprises a pigment chlorophyll, preferably said chlorophyll is chlorophyll A;
l. said sodium chloride resistant microalgae biomass comprises an antibacterial substance chlorellin;
m. said sodium chloride resistant microalgae biomass comprises phycocyanin;
n. said sodium chloride resistant microalgae biomass comprises arachidonic acid (ARA), preferably said biomass is a producer of arachidonic acid (ARA);
o. said sodium chloride resistant microalgae biomass comprises an acidic polysaccharide Chlon A;
p. cells of said sodium chloride resistant microalgae biomass have no nuclei;
q. said microalgae biomass does not comprise bacteriorhodopsin.

5. A composition comprising sodium chloride and sodium chloride resistant microalgae biomass selected from the group consisting of:Arthrospira (Spirulina) maxima designated strain Absheron Accession No. BEA D03_12, Chlorella vulgaris designated strain Novruz 2012 Accession No. BEA D02_12, and mixtures thereof produced by the process of claims 1-4.

6. A dry composition comprising: a) a carrier comprising sodium chloride and b) a sodium chloride resistant microalgae biomass selected from the group consisting of: *Arthrospira (Spirulina) maxima* designated strain Absheron Accession No. BEA D03_12, *Chlorella vulgaris* designated strain Novruz 2012 Accession No. BEA D02_12, and mixtures thereof, wherein at least 75% of cells of said sodium chloride resistant microalgae biomass remain whole in said composition.

7. The composition of claim 6, wherein said composition has one or more of the following features:
a. cells of said sodium chloride resistant microalgae biomass are non-flagellated;
b. cells of said sodium chloride resistant microalgae biomass are non-cyst-forming;
c. cells of said sodium chloride resistant microalgae biomass are non-motile, preferably said non-motile cells are free-floating;
d. cells of said sodium chloride resistant microalgae biomass have a cell wall, preferably a rigid cell wall, further preferably a rigid cell wall comprising cellulose;
e. said sodium chloride resistant microalgae biomass is capable of growing at sodium chloride concentration in the range of 0.1 - 1 M, preferably in the range of 0.1 - 0.75 M; further preferably in the range of 0.1 - 0.5 M;
f. said sodium chloride resistant microalgae biomass is capable of growing in the temperature range of 0°C - 45°C, preferably 10°C - 45°C, further preferably in the temperature range of 20°C - 45°C;
g. said sodium chloride resistant microalgae biomass is capable of growing in the pH range of 6.0-14.0 or in the pH range of 5.0-9.0;
h. said sodium chloride resistant microalgae biomass has a total concentration of polyunsaturated fatty acids of at least 2% of its dry weight;
i. said sodium chloride resistant microalgae biomass has a concentration in said composition of 1-2% of dry weight;
j. said living sodium chloride resistant microalgae biomass has a productivity of at least 5 g/m², preferably at least 10 g/m², further preferably at least 20 g/m², further preferably at least 40 g/m², further preferably at least 80 g/m²;
k. said sodium chloride resistant microalgae biomass comprises a pigment chlorophyll, preferably said chlorophyll is chlorophyll A;
l. said sodium chloride resistant microalgae biomass comprises an antibacterial substance chlorellin;
m. said sodium chloride resistant microalgae biomass comprises phycocyanin;
n. said sodium chloride resistant microalgae biomass comprises arachidonic acid (ARA), preferably said biomass is a producer of arachidonic acid (ARA);
o. said sodium chloride resistant microalgae biomass comprises an acidic polysaccharide Chlon A;
p. cells of said sodium chloride resistant microalgae biomass have no nuclei;
q. said microalgae biomass does not comprise bacteriorhodopsin.

8. The composition of claims 5-7 for use as a medicament.

9. The composition of claims 5-8 for use in prevention and/or treatment of a disease selected from the group consisting of: vitamin deficiency, iron-deficiency anemia, megaloblastic anemia, pernicious anemia, intestinal ulcers, diverticulosis, Crohn's disease, hypertension, cancer, diabetes, arthritis, AIDS, pancreatitis, cirrhosis, hepatitis, anemia, and multiple sclerosis.

10. Use of the composition of claims 5-7 in preparation of a foodstuff or a fodder, or an intermediate foodstuff or an intermediate fodder, or a nutritional supplement, or a prebiotic, or a pharmaceutical composition, or a seasoning, or a spice or a mixture thereof.

11. The composition of claims 5-7 for use in the detoxification or antioxidant treatment.

12. A foodstuff or a fodder, or an intermediate foodstuff or an intermediate fodder, or a nutritional supplement, or a prebiotic, or a pharmaceutical composition, or a seasoning, or a spice, or a detoxifying agent, or an antioxidant comprising the composition of claims 5-7.

13. Non-flagellated, non-cyst-forming sodium chloride resistant microalgae, wherein said sodium chloride resistant microalgae is selected from the group consisting of: *Arthrospira (Spirulina) maxima* designated strain Absheron Accession No. BEA D03_12 and *Chlorella vulgaris* designated strain Novruz 2012 Accession No. BEA D02_12.

## Patentansprüche

1. Verfahren zur Herstellung einer Zusammensetzung, die Natriumchlorid und Natriumchlorid-resistente Mikroalgenbiomasse umfasst, wobei das Verfahren umfasst:
a) Bereitstellung:
i) eines Trägers, der Natriumchlorid umfasst;
ii) einer lebenden Natriumchlorid-resistenten Mikroalgenbiomasse, ausgewählt aus der Gruppe bestehend aus: dem Arthrospira (Spirulina) maxima designierten Stamm Absheron mit Zugangsnummer BEA D03_12, dem Chlorella vulgaris designierten Stamm Novruz 2012 mit Zugangsnummer BEA D02_12 und Mischungen davon, wobei besagte lebende Natriumchlorid-resistente Mikroalgenbiomasse vorzugsweise frisch gewachsen ist, wobei weiter vorzugsweise die lebende Natriumchlorid-resistente Mikroalgenbiomasse auf einem den Träger als Natriumchloridquelle enthaltenden Wachstumsmedium frisch gewachsen ist, und
b) Mischen der lebenden Natriumchlorid-resistenten Mikroalgenbiomasse mit dem besagten Träger, wobei das Mischen mindestens drei Stufen umfasst, wobei jede folgende Stufe eine höhere Natriumchloridkonzentration als eine vorherige Stufe aufweist;
c) Trocknung der besagten Mischung, wobei besagte Trocknung vorzugsweise unter Lichtschutzbedingungen durchgeführt wird, wobei weiter vorzugsweise die Lichtschutzbedingungen eine Beleuchtungsstärke von weniger als 1000 Lux aufweisen, und wobei mindestens 75 % der Zellen der Natriumchlorid-resistenten Mikroalgenbiomasse nach dem Trocknen ganz geblieben sind.

2. Verfahren nach Anspruch 1, wobei die lebende Natriumchlorid-resistente Mikroalgenbiomasse auf einem Wachstumsmedium wächst, das den Träger als Quelle für Natriumchlorid umfasst.

3. Verfahren nach den Ansprüchen 1-2, wobei 99 % der Zellen der besagten Natriumchlorid-resistenten Mikroalgenbiomasse nach dem Trocknen ganz geblieben sind.

4. Verfahren nach den Ansprüchen 1-3, wobei die besagte Zusammensetzung eines oder mehrere der folgenden Merkmale aufweist:
a. die Zellen der Natriumchlorid-resistenten Mikroalgenbiomasse weisen keine Geißeln auf;
b. die Zellen der Natriumchlorid-resistenten Mikroalgenbiomasse bilden keine Zysten;
c. die Zellen der Natriumchlorid-resistenten Mikroalgenbiomasse sind nicht beweglich, vorzugsweise sind die nicht beweglichen Zellen frei schwebend;
d. die Zellen der Natriumchlorid-resistenten Mikroalgenbiomasse haben eine Zellwand, vorzugsweise eine starre Zellwand, ferner vorzugsweise eine starre Zellwand, die Zellulose enthält;
e. die Natriumchlorid-resistente Mikroalgenbiomasse kann bei einer Natriumchloridkonzentration im Bereich von 0,1 - 1 M, vorzugsweise im Bereich von 0,1 - 0,75 M, weiter bevorzugt im Bereich von 0,1 - 0,5 M wachsen;
f. die Natriumchlorid-resistente Mikroalgenbiomasse kann im Temperaturbereich von 0°C - 45°C, vorzugsweise 10°C - 45°C, weiterhin vorzugsweise im Temperaturbereich von 20°C - 45°C wachsen;
g. die Natriumchlorid-resistente Mikroalgenbiomasse kann im pH-Bereich von 6,0-14,0 oder im pH-Bereich von 5,0-9,0 wachsen;
h. nach dem Trocknen hat die Natriumchlorid-resistenten Mikroalgenbiomasse eine Gesamtkonzentration an mehrfach ungesättigten Fettsäuren von mindestens 2% ihres Trockengewichts;
i. nach dem Trocknen hat die Natriumchlorid-resistenten Mikroalgenbiomasse in der Zusammensetzung eine Konzentration von 1-2% des Trockengewichts;
j. die lebende Natriumchlorid-resistente Mikroalgenbiomasse hat eine Produktivität von mindestens 5 g/m2, vorzugsweise mindestens 10 g/m2, weiter vorzugsweise mindestens 20 g/m2, weiter vorzugsweise mindestens 40 g/m2, weiter vorzugsweise mindestens 80 g/m2;
k. die Natriumchlorid-resistente Mikroalgenbiomasse enthält Chlorophyll als Pigment, vorzugsweise ist besagtes Chlorophyll Chlorophyll A;
l. die Natriumchlorid-resistente Mikroalgenbiomasse enthält als antibakterielle Substanz Chlorellin;
m. die Natriumchlorid-resistente Mikroalgenbiomasse enthält Phycocyanin;
n. die Natriumchlorid-resistente Mikroalgenbiomasse umfasst Arachidonsäure (ARA), vorzugsweise ist die Biomasse ein Produzent von Arachidonsäure (ARA);
o. die Natriumchlorid-resistente Mikroalgenbiomasse umfasst als saures Polysaccharid Chlon A;
p. die Zellen der Natriumchlorid-resistenten Mikroalgenbiomasse weisen keine Kerne auf;
q. die Mikroalgenbiomasse umfasst kein Bakteriorhodopsin.

5. Zusammensetzung, umfassend Natriumchlorid und Natriumchlorid-resistente Mikroalgenbiomasse, ausgewählt aus der Gruppe bestehend aus: dem Arthrospira (Spirulina) maxima designierten Stamm Absheron mit Zugangsnummer BEA D03_12, dem Chlorella vulgaris designierten Stamm Novruz 2012 mit Zugangsnummer BEA D02_12 und Mischungen davon, hergestellt nach dem Verfahren der Ansprüche 1-4.

6. Trockene Zusammensetzung, umfassend: a) einen Träger, umfassend Natriumchlorid und b) eine Natriumchlorid-resistente Mikroalgenbiomasse, ausgewählt aus der Gruppe bestehend aus:
dem Arthrospira (Spirulina) maxima designierten Stamm Absheron mit Zugangsnummer BEA D03_12, dem Chlorella vulgaris designierten Stamm Novruz 2012 mit Zugangsnummer BEA D02_12 und Mischungen davon, wobei mindestens 75% der Zellen der Natriumchlorid-resistenten Mikroalgenbiomasse in der Zusammensetzung ganz geblieben sind.

7. Zusammensetzung nach Anspruch 6, wobei die besagte Zusammensetzung eines oder mehrere der folgenden Merkmale aufweist:
a. die Zellen der Natriumchlorid-resistenten Mikroalgenbiomasse weisen keine Geißeln auf;
b. die Zellen der Natriumchlorid-resistenten Mikroalgenbiomasse bilden keine Zysten;
c. die Zellen der Natriumchlorid-resistenten Mikroalgenbiomasse sind nicht beweglich, vorzugsweise sind die nicht beweglichen Zellen frei schwebend;
d. die Zellen der Natriumchlorid-resistenten Mikroalgenbiomasse haben eine Zellwand, vorzugsweise eine starre Zellwand, ferner vorzugsweise eine starre Zellwand, die Zellulose enthält;
e. die Natriumchlorid-resistente Mikroalgenbiomasse kann bei einer Natriumchloridkonzentration im Bereich von 0,1 - 1 M, vorzugsweise im Bereich von 0,1 - 0,75 M, weiter bevorzugt im Bereich von 0,1 - 0,5 M wachsen;
f. die Natriumchlorid-resistente Mikroalgenbiomasse kann im Temperaturbereich von 0°C - 45°C, vorzugsweise 10°C - 45°C, weiterhin vorzugsweise im Temperaturbereich von 20°C - 45°C wachsen;
g. die Natriumchlorid-resistente Mikroalgenbiomasse kann im pH-Bereich von 6,0-14,0 oder im pH-Bereich von 5,0-9,0 wachsen;
h. nach dem Trocknen hat die Natriumchlorid-resistenten Mikroalgenbiomasse eine Gesamtkonzentration an mehrfach ungesättigten Fettsäuren von mindestens 2% ihres Trockengewichts;
i. die Natriumchlorid-resistenten Mikroalgenbiomasse hat in der Zusammensetzung eine Konzentration von 1-2% des Trockengewichts;
j. die lebende Natriumchlorid-resistente Mikroalgenbiomasse hat eine Produktivität von mindestens 5 g/m2, vorzugsweise mindestens 10 g/m2, weiter vorzugsweise mindestens 20 g/m2, weiter vorzugsweise mindestens 40 g/m2, weiter vorzugsweise mindestens 80 g/m2;
k. die Natriumchlorid-resistente Mikroalgenbiomasse enthält Chlorophyll als Pigment, vorzugsweise ist besagtes Chlorophyll Chlorophyll A;
l. die Natriumchlorid-resistente Mikroalgenbiomasse enthält als antibakterielle Substanz Chlorellin;
m. die Natriumchlorid-resistente Mikroalgenbiomasse enthält Phycocyanin;
n. die Natriumchlorid-resistente Mikroalgenbiomasse umfasst Arachidonsäure (ARA), vorzugsweise ist die Biomasse ein Produzent von Arachidonsäure (ARA);
o. die Natriumchlorid-resistente Mikroalgenbiomasse umfasst als saures Polysaccharid Chlon A;
p. die Zellen der Natriumchlorid-resistenten Mikroalgenbiomasse weisen keine Kerne auf;
q. die Mikroalgenbiomasse umfasst kein Bakteriorhodopsin.

8. Zusammensetzung nach den Ansprüchen 5-7 zur Verwendung als Medikament.

9. Zusammensetzung nach den Ansprüchen 5-8 zur Verwendung bei der Vorbeugung und/oder Behandlung einer Krankheit, ausgewählt aus der Gruppe bestehend aus: Vitaminmangel, Eisenmangelanämie, megaloblastischer Anämie, perniziöser Anämie, Darmgeschwüren, Divertikulose, Morbus Crohn, Bluthochdruck, Krebs, Diabetes, Arthritis, AIDS, Pankreatitis, Zirrhose, Hepatitis, Anämie und Multipler Sklerose.

10. Verwendung der Zusammensetzung nach den Ansprüchen 5-7 bei der Herstellung eines Lebensmittels oder eines Futtermittels, eines Zwischenprodukts eines Lebensmittels oder eines Futtermittels oder eines Nahrungsergänzungsmittels oder eines Präbiotikums oder einer pharmazeutischen Zusammensetzung oder eines Würzmittels oder eines Gewürzes oder einer Mischung davon.

11. Zusammensetzung nach den Ansprüchen 5-7 zur Verwendung bei einer Entgiftungs- oder Antioxidationsbehandlung.

12. Lebensmittel oder Futtermittel oder Zwischenprodukt eines Lebensmittels oder eines Futtermittels oder Nahrungsergänzungsmittel oder Präbiotikum oder pharmazeutischen Zusammensetzung oder Würzmittel oder Gewürz oder Entgiftungsmittel oder Antioxidationsmittel, umfassend die Zusammensetzung nach den Ansprüchen 5-7 umfasst.

13. Natriumchlorid-resistente Mikroalgen, die keine Geißeln aufweisen und keine Zysten bilden, wobei die Natriumchlorid-resistente Mikroalge ausgewählt ist aus der Gruppe bestehend aus: dem Arthrospira (Spirulina) maxima designierten Stamm Absheron mit Zugangsnummer BEA D03_12 und dem Chlorella vulgaris designierten Stamm Novruz 2012 mit Zugangsnummer BEA D02_12.

## Revendications

1. Procédé de préparation d'une composition comprenant du chlorure de sodium et une biomasse de microalgues résistantes au chlorure de sodium, ledit procédé comprenant :
a) la fourniture
i) d'un véhicule comprenant du chlorure de sodium ;
ii) d'une biomasse vivante de microalgues résistantes au chlorure de sodium choisies dans le groupe constitué par la souche désignée par Arthrospira (Spirulina) maxima Absheron de n° d'accès BEA D03_12, la souche désignée par Chlorella vulgaris Novruz 2012 de n° d'accès BEA D02_12, et des mélanges de celles-ci, préférablement où ladite biomasse vivante de microalgues résistantes au chlorure de sodium est fraîchement cultivée, en outre préférablement ladite biomasse vivante de microalgues résistantes au chlorure de sodium est fraîchement cultivée sur un milieu de culture comprenant ledit véhicule comme source de chlorure de sodium, et
b) le mélange de ladite biomasse vivante de microalgues résistantes au chlorure de sodium avec ledit véhicule, ledit mélange comprenant au moins trois étapes, où chaque étape suivante possède une concentration en chlorure de sodium supérieure par rapport à une étape précédente ;
c) le séchage dudit mélange, préférablement ledit séchage étant effectué dans des conditions de protection contre la lumière, en outre préférablement lesdites conditions de protection contre la lumière possèdent un éclairement inférieur à 1000 lux, et où au moins 75% des cellules de ladite biomasse de microalgues résistantes au chlorure de sodium restent entières après séchage.

2. Procédé selon la revendication 1, dans lequel ladite biomasse vivante de microalgues résistantes au chlorure de sodium est cultivée sur un milieu de croissance comprenant ledit véhicule comme source de chlorure de sodium.

3. Procédé selon les revendications 1-2, dans lequel 99% des cellules de ladite biomasse de microalgues résistantes au chlorure de sodium restent entières après séchage.

4. Procédé selon les revendications 1-3, dans lequel ladite composition possède une ou plusieurs parmi les caractéristiques suivantes :
a. les cellules de ladite biomasse de microalgues résistantes au chlorure de sodium sont non flagellées ;
b. les cellules de ladite biomasse de microalgues résistantes au chlorure de sodium ne sont pas formatrices de sporocystes ;
c. les cellules de ladite biomasse de microalgues résistantes au chlorure de sodium ne sont pas vagiles, préférablement lesdites cellules non vagiles flottent librement ;
d. les cellules de ladite biomasse de microalgues résistantes au chlorure de sodium possèdent une paroi cellulaire, préférablement une paroi cellulaire rigide, en outre préférablement une paroi cellulaire rigide comprenant de la cellulose ;
e. ladite biomasse de microalgues résistantes au chlorure de sodium est capable de croître à une concentration en chlorure de sodium dans la plage de 0,1-1M, préférablement dans la plage de 0,1-0,75M, en outre préférablement dans la plage de 0,1-0,5M ;
f. ladite biomasse de microalgues résistantes au chlorure de sodium est capable de croître dans une plage de température de 0°C-45°C, préférablement de 10°C-45°C, en outre préférablement dans une plage de température de 20°C-45°C ;
g. ladite biomasse de microalgues résistantes au chlorure de sodium est capable de croître dans une plage de pH de 6,0-14,0 ou dans une plage de pH de 5,0-9,0 ;
h. après séchage, ladite biomasse de microalgues résistantes au chlorure de sodium possède une concentration totale en acides gras polyinsaturés d'au moins 2% de son poids sec ;
i. après séchage, ladite biomasse de microalgues résistantes au chlorure de sodium possède une concentration dans ladite composition de 1-2% de poids sec ;
j. ladite biomasse vivante de microalgues résistantes au chlorure de sodium possède une productivité d'au moins 5 g/m2, préférablement d'au moins 10 g/m2, en outre préférablement d'au moins 20 g/m2, en outre préférablement d'au moins 40 g/m2, en outre préférablement d'au moins 80 g/m2 ;
k. ladite biomasse de microalgues résistantes au chlorure de sodium comprend une chlorophylle pigmentaire, préférablement ladite chlorophylle est la chlorophylle A ;
l. ladite biomasse de microalgues résistantes au chlorure de sodium comprend une substance antibactérienne, la chlorelline ;
m. ladite biomasse de microalgues résistantes au chlorure de sodium comprend de la phycocyanine ;
n. ladite biomasse de microalgues résistantes au chlorure de sodium comprend de l'acide arachidonique (ARA), préférablement ladite biomasse est un producteur d'acide arachidonique (ARA) ;
o. ladite biomasse de microalgues résistantes au chlorure de sodium comprend un polysaccharide acide, le Chlon A ;
p. les cellules de ladite biomasse de microalgues résistantes au chlorure de sodium ne possèdent pas de noyau ;
q. ladite biomasse de microalgues ne comprend pas de bactériorhodopsine.

5. Composition comprenant du chlorure de sodium et une biomasse de microalgues résistantes au chlorure de sodium choisies dans le groupe constitué par la souche désignée par Arthrospira (Spirulina) maxima Absheron de n° d'accès BEA D03_12, la souche désignée par Chlorella vulgaris Novruz 2012 de n° d'accès BEA D02_12, et des mélanges de celles-ci, produite par le procédé selon les revendications 1-4.

6. Composition sèche, comprenant : a) un véhicule comprenant du chlorure de sodium et b) une biomasse de microalgues résistantes au chlorure de sodium choisies dans le groupe constitué par la souche désignée par Arthrospira (Spirulina) maxima Absheron de n° d'accès BEA D03_12, la souche désignée par Chlorella vulgaris Novruz 2012 de n° d'accès BEA D02_12, et des mélanges de celles-ci, où au moins 75% des cellules de ladite biomasse de microalgues résistantes au chlorure de sodium restent entières dans ladite composition.

7. Composition selon la revendication 6, où ladite composition possède une ou plusieurs parmi les caractéristiques suivantes :
a. les cellules de ladite biomasse de microalgues résistantes au chlorure de sodium sont non flagellées ;
b. les cellules de ladite biomasse de microalgues résistantes au chlorure de sodium ne sont pas formatrices de sporocystes ;
c. les cellules de ladite biomasse de microalgues résistantes au chlorure de sodium ne sont pas vagiles, préférablement lesdites cellules non vagiles flottent librement ;
d. les cellules de ladite biomasse de microalgues résistantes au chlorure de sodium possèdent une paroi cellulaire, préférablement une paroi cellulaire rigide, en outre préférablement une paroi cellulaire rigide comprenant de la cellulose ;
e. ladite biomasse de microalgues résistantes au chlorure de sodium est capable de croître à une concentration en chlorure de sodium dans la plage de 0,1-1M, préférablement dans la plage de 0,1-0,75M, en outre préférablement dans la plage de 0,1-0,5M ;
f. ladite biomasse de microalgues résistantes au chlorure de sodium est capable de croître dans une plage de température de 0°C-45°C, préférablement de 10°C-45°C, en outre préférablement dans une plage de température de 20°C-45°C ;
g. ladite biomasse de microalgues résistantes au chlorure de sodium est capable de croître dans une plage de pH de 6,0-14,0 ou dans une plage de pH de 5,0-9,0 ;
h. ladite biomasse de microalgues résistantes au chlorure de sodium possède une concentration totale en acides gras polyinsaturés d'au moins 2% de son poids sec ;
i. ladite biomasse de microalgues résistantes au chlorure de sodium possède une concentration dans ladite composition de 1-2% de poids sec ;
j. ladite biomasse vivante de microalgues résistantes au chlorure de sodium possède une productivité d'au moins 5 g/m2, préférablement d'au moins 10 g/m2, en outre préférablement d'au moins 20 g/m2, en outre préférablement d'au moins 40 g/m2, en outre préférablement d'au moins 80 g/m2 ;
k. ladite biomasse de microalgues résistantes au chlorure de sodium comprend une chlorophylle pigmentaire, préférablement ladite chlorophylle est la chlorophylle A ;
l. ladite biomasse de microalgues résistantes au chlorure de sodium comprend une substance antibactérienne, la chlorelline ;
m. ladite biomasse de microalgues résistantes au chlorure de sodium comprend de la phycocyanine ;
n. ladite biomasse de microalgues résistantes au chlorure de sodium comprend de l'acide arachidonique (ARA), préférablement ladite biomasse est un producteur d'acide arachidonique (ARA) ;
o. ladite biomasse de microalgues résistantes au chlorure de sodium comprend un polysaccharide acide, le Chlon A ;
p. les cellules de ladite biomasse de microalgues résistantes au chlorure de sodium ne possèdent pas de noyau ;
q. ladite biomasse de microalgues ne comprend pas de bactériorhodopsine.

8. Composition selon les revendications 5-7, pour une utilisation comme médicament.

9. Composition selon les revendications 5-8, pour une utilisation dans la prévention et/ou le traitement d'une maladie choisie dans le groupe constitué par la déficience en vitamines, l'anémie liée à une déficience en fer, l'anémie mégaloblastique, l'anémie pernicieuse, les ulcères intestinaux, la diverticulose, la maladie de Crohn, l'hypertension, le cancer, le diabète, l'arthrite, le SIDA, la pancréatite, la cirrhose, l'hépatite, l'anémie, et la sclérose en plaques.

10. Utilisation de la composition selon les revendications 5-7, dans la préparation d'un produit alimentaire ou d'un fourrage, ou d'un produit alimentaire intermédiaire ou d'un fourrage intermédiaire, ou d'un complément nutritionnel, ou d'un prébiotique, ou d'une composition pharmaceutique, ou d'un assaisonnement, ou d'une épice, ou d'un mélange de ceux-ci.

11. Composition selon les revendications 5-7, pour une utilisation dans la détoxification ou un traitement antioxydant.

12. Produit alimentaire ou fourrage, ou produit alimentaire intermédiaire ou fourrage intermédiaire, ou complément nutritionnel, ou prébiotique, ou composition pharmaceutique, ou assaisonnement, ou épice, ou agent détoxifiant, ou antioxydant, comprenant la composition selon les revendications 5-7.

13. Microalgues résistantes au chlorure de sodium non flagellées et non formatrices de sporocystes, où lesdites microalgues résistantes au chlorure de sodium sont choisies dans le groupe constitué par la souche désignée par Arthrospira (Spirulina) maxima Absheron de n° d'accès BEA D03_12 et la souche désignée par Chlorella vulgaris Novruz 2012 de n° d'accès BEA D02_12.
